# EUROPÄISCHE PATENTANMELDUNG

(11) **EP 1 010 394 A1**
(43) Veröffentlichungstag der Anmeldung: **21.06.2000**
(21) Anmeldenummer: 99123754.6
(22) Anmeldetag: 30.11.1999
(51) Int. Cl.: A61B 10/00, A61M 31/00

(54) **Endoskopisches Instrument zur Entnahme von Körperflüssigkeiten**

(30) Priorität: 16.12.1998 AT 83498
(71) Anmelder: AMI (Agency for medical innovations GmbH), 6840 GötzisAT (AT)
(72) Erfinder: Höfle, Siegried, Dipl.-Ing., 6840 Götzis (AT)
(74) Vertreter: Riebling, Peter, Dr.-Ing.

(57) **Zusammenfassung**

Die Erfindung betrifft ein endoskopisches Instrument zur Entnahme von Gewebsflüssigkeiten, welches auswechselbar am vorderen Ende eines Applikationsrohres angeordnet ist und eine Kolbenstange (8) aufweist, die mit einer entsprechenden Zugstange eines endoskopischen Applikators koppelbar ist. Durch eine Kappe (17) abdeckbare Saugöffnungen (16) können sowohl an der Vorderseite der Zylinderkammer (15) vorgesehen sein, als auch seitlich im vorderen Zylindermantel, jedoch immer in distaler Richtung vor dem Kolben (9). Dieses endoskopische Instrument kann auch Flüssigkeiten über die Saugöffnungen durch Zurückbewegen der Zugstange und damit der Kolbenstange mit dem Kolben (9) abgeben.

## Beschreibung

Die Erfindung betrifft ein endoskopisches Instrument zur Entnahme von Gewebsflüssigkeiten nach dem Oberbegriff des Schutzanspruches 1. Endoskopische Instrumente werden zum Einführen in Körperöffnungen verwendet, um am Operationsort mikrochirurgische Behandlungen, Operationen oder Untersuchungen durchzuführen.

Mit dem Gegenstand US 5 312 333 ist bereits schon ein endoskopischer Applikator bekannt geworden, bei dem in einem Applikationsrohr eine Kolbenstange verschiebbar ausgebildet ist, welche in der Lage ist, einen vor der Kolbenstange angeordneten Tupfer aus dem Applikationsrohr herauszuschieben.

Nachteil dieser bekannten Anordnung ist jedoch, daß sie nicht ohne weiteres auswechselbar ist, denn zur Auswechslung muß das gesamte, relativ lang ausgebildete Applikationsrohr ausgewechselt werden.

Weiterer Nachteil ist, daß eine derartige Anordnung nicht zur Entnahme von Gewebsflüssigkeiten oder Körperflüssigkeiten aus dem Körper geeignet ist, denn die dort gezeigte Kolbenstange dient nur als mechanische Ausstoßhilfe, nicht jedoch zur Aufnahme oder zur Abgabe von Flüssigkeiten.

Der Erfindung liegt deshalb die Aufgabe zugrunde, einen endoskopischen Applikator so weiter zu bilden, daß er zur Entnahme von Gewebsflüssigkeiten bzw. zur Abgabe von Flüssigkeiten geeignet ist.

Zur Lösung der gestellten Aufgabe ist die Erfindung durch die technische Lehre des Anspruches 1 gekennzeichnet.

Wesentliches Merkmal der Erfindung ist, daß nun ein endoskopisches Instrument auswechselbar am vorderen Ende eines Applikationsrohres angeordnet ist und daß dieses endoskopische Instrument eine Kolbenstange aufweist, die mit einer entsprechenden Zugstange eines endoskopischen Applikators koppelbar ist, so daß mit der Betätigung der Zugstange im Handgriff des Applikators die Kolbenstange des Instrumentes betätigt wird, und der Kolben hierdurch in eine Zylinderkammer verschoben wird.

Mit der entsprechenden Verschiebung des Kolbens in der Zylinderkammer ist es deshalb möglich, sowohl Flüssigkeiten aus der Zylinderkammer in das umgebende Gewebe über die Stirnseite der Zylinderkammer auszugeben, als auch entsprechende Gewebsflüssigkeiten oder Körperflüssigkeiten über einen an der Vorderseite der Zylinderkammer angeordneten Deckel, der mit Saugöffnungen ausgebildet ist, in die Zylinderkammer aufzunehmen.

Die Erfindung ist jedoch nicht auf die Anordnung von Saugöffnungen an der Stirnseite der Zylinderkammer begrenzt. Es ist in einer anderen Ausgestaltung vorgesehen, daß die Zylinderkammer seitlich entsprechende Saugöffnungen aufweist. Selbstverständlich müssen die Saugöffnungen immer in distaler Richtung vor dem Kolben sitzen, welcher fest mit der Kolbenstange verbunden ist, um in diesen Teil der Zylinderkammer entsprechende Flüssigkeiten aufzunehmen oder abzugeben.

Ein weiteres wesentliches Merkmal der Erfindung ist, daß die gesamte Vorderseite der Zylinderkammer mit dem dort bündig eingelassenen Deckel mittels einer Kappe verschließbar ist, um so den Inhalt der Zylinderkammer gegen Auslaufen zu schützen. Wichtig ist, daß das gesamte endoskopische Instrument als Einmalinstrument ausgebildet ist, weil es leicht lösbar an einem Applikationsrohr eines endoskopischen Applikators befestigt ist. Damit ergibt sich der Vorteil, daß man jeweils bei einem gleichbleibenden endoskopischen Applikator, der nicht ausgewechselt werden muß, stets ein Einmal-Instrument zur Verfügung hat, welches leicht auswechselbar dort gehalten ist.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung, offenbarten Angaben und Merkmale, insbesondere die in den Zeichnungen dargestellte räumliche Ausbildung werden als erfindungswesentlich beansprucht, soweit sie einzeln oder in Kombination gegenüber dem Stand der Technik neu sind.

Im folgenden wird die Erfindung anhand von lediglich einem Ausführungsweg der darstellenden Zeichnungen näher erläutert. Hierbei gehen aus den Zeichnungen und ihrer Beschreibung weitere erfindungswesentliche Merkmale und Vorteile der Erfindung hervor.

Es zeigen:

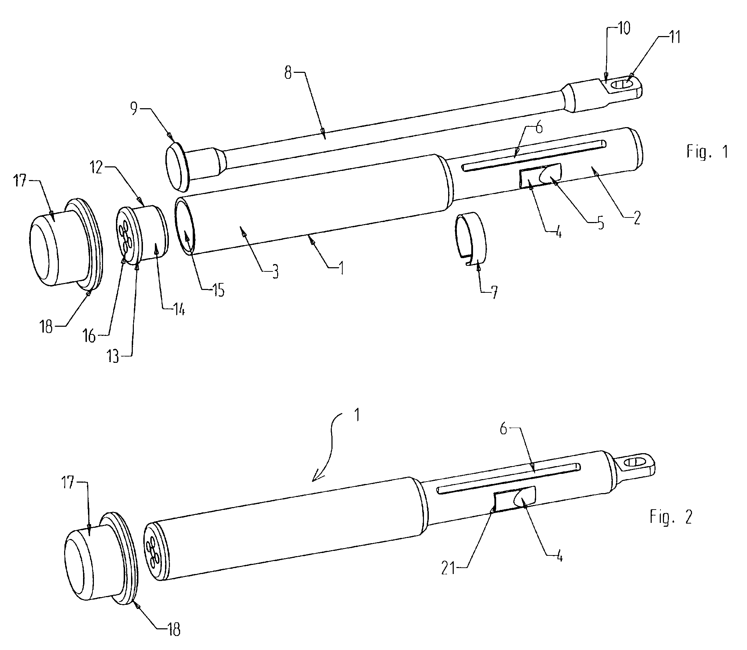

- Figur 1:: Explosionsartige Darstellung eines endoskopischen Instrumentes nach der Erfindung
- Figur 2:: Das Instrument nach Fig. 1 in zusammengebautem Zustand
- Figur 3:: Schnitt durch das Instrument nach Fig. 2
- Figur 4:: Ein um 90° gedrehter Schnitt nach Fig. 3
- Figur 5:: Schnitt gemäß der Linie V - V in Fig. 3

Das endoskopische Instrument nach der Erfindung besteht im wesentlichen nach dem Grundkörper 1, der aus zwei hintereinanderliegenden Hohlzylindern unterschiedlicher Durchmessers besteht. Der Grundkörper ist bevorzugt aus einem kostengünstigen Kunststoffmaterial im Spritzgußverfahren hergestellt. Das Kunststoffmaterial ist mit Körperflüssigkeit biologisch verträglich.

Der kleinere Zylinder des Grundkörpers 1 ist als Halterungsschaft 2 ausgebildet, der geeignet ist, in eine zugeordnete Aufnahmeöffnung eines nicht näher dargestellten Applikationsrohres, eines endoskopischen Applikators eingesteckt und dort gehalten zu werden.

Es ist nicht dargestellt, daß in diesem Applikationsrohr ein Rastfenster vorhanden ist, welches etwas größer ausgebildet ist, als die am Halterungsschaft angeordnete Rastnase, so daß beim Einstecken des Halterungsschaftes 2 in dieses Applikationsrohr die Rastnase 4 in das Rastfenster eingreift und dort mit einer Rastkante 21 einrastet.

Der Halterungsschaft 2 ist damit gegen unbeabsichtigtes Herausziehen aus dem Applikationsrohr des endoskopischen Applikators geschützt

An dem Halterungsschaft 2 schließt sich werkstoffeinstückig ein Zylinder größeren Durchmessers an, der als Zylinderkörper 3 ausgebildet ist und der eine Zylinderkammer 15 aufweist, in der längsverschieblich ein Kolben 9 verschiebbar ist, der fest mit einer Kolbenstange 8 verbunden ist.

Die Kolbenstange 8 greift also in den Zylinderkörper 3 hinein, greift durch den Halterungsschaft 2 hindurch und ragt auf dem hinteren Ende des Halterungsschaftes 2 in Form einer Einhängung 10 heraus, welche mit einer Öse 11 verbunden ist.

In diese Öse 11 greift ein hakenförmiges Teil, welches mit einer Zugstange fest verbunden ist, die in dem nicht näher dargestellten endoskopischen Applikator in dem vorher erwähnten Applikationsrohr längsverschieblich geführt ist.

Mit der Betätigung der Zugstange, wird damit die Kolbenstange 8 in dem Grundkörper 1 längsverschieblich verschoben und der Kolben 9 gleitet damit in der Zylinderkammer 15 hin und her.

Es wird noch darauf hingewiesen, daß am Ende der Zylinderkammer ein Ring 7 eingelassen ist, der am Innenumfang anliegt, welcher Ring aus einem röntgendichten Material besteht, so daß der gesamte Grundkörper durch eine Röntgenaufnahme im Körper erkennbar ist, wenn er verlorengehen sollte.

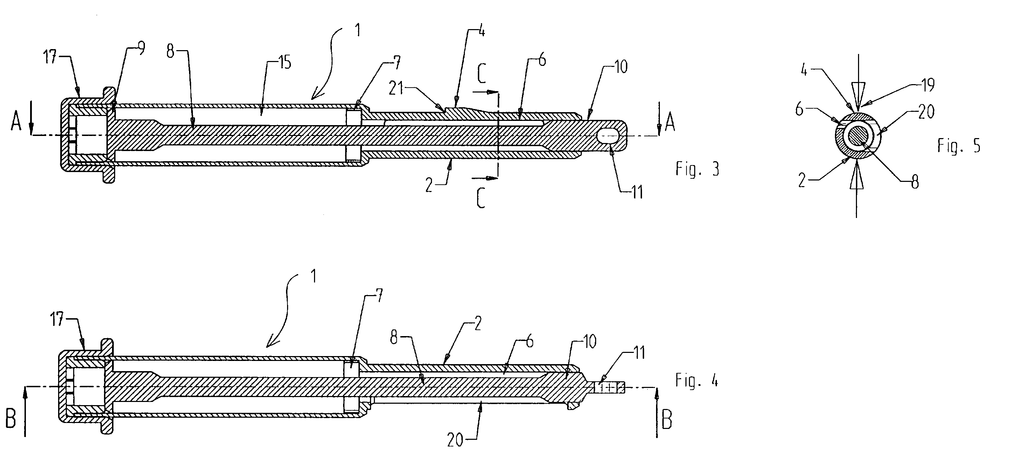

Zur Betätigung der Rastnase 4 ist im übrigen ein Schlitz 6 im Halterungsschaft 2 angeordnet, dessen Funktion anhand der Figur 5 noch näher beschrieben wird.

Zum erleichternden Einrasten der Rastnase 4 in das nicht näher dargestellte Rastfenster des Applikationsrohres weist die Rastnase 4 an ihrem proximalen Ende eine Anschrägung 5 auf die als Gleitfläche ausgebildet ist, so daß also zunächst die Gleitfläche auf den Innenumfang des Applikationsrohres trifft, hierbei die Rastnase 4 radial einwärts nach innen verformt wird und sobald die Rastnase 4 in Gegenüberstellung zu dem nicht näher dargestellten Rastfenster gelangt, rastet diese aus und die Rastkante 21 legt sich an einer zugeordneten Kante des Rastfensters an.

Das vordere Ende der Zylinderkammer 15 wird durch einen Deckel 12 verschlossen, der im wesentlichen aus einem Schaft 14 geringeren Durchmessers besteht, der passend in der Zylinderkammer 15 einführbar ist, welcher Schaft 14 nach vorne durch eine Scheibe 13 größeren Durchmessers abgeschlossen ist und werkstoffeinstückig mit dieser verbunden ist. Die Scheibe 13 ist durch eine Anzahl von Saugöffnungen 16 durchbrochen.

Die gesamte Anordnung kann durch eine Kappe 17 dichtend abgeschlossen werden.

Hierzu kann die Kappe 17 mit einem Rand 18 vergrößerten Durchmessers ergriffen und auf dem Deckel 12 dichtend aufgesetzt werden. Dies ist in Figur 3 und 4 dargestellt.

Um der Rastnase 4 nun ein entsprechendes, radial einwärts gerichtetes elastisches Verformungsvermögen zu geben, ist ein Schlitz 6 in dem Haltungsschaft 2 eingeformt, der dafür sorgt, daß die Seitenwandungen, in deren Bereich sich die Rastnase 4 befindet in Pfeilrichtung 19 zusammengedrückt werden können, weil der Schlitz 6 ein entsprechendes radial einwärts gerichtetes Bewegungsspiel zuläßt, wie dies die Figur 5 darstellt.

Figur 5 zeigt im übrigen, daß an der Unterseite des Halterungsschaftes 2 eine in Längsrichtung verlaufende Entformungsöffnung 20 vorhanden ist, durch welche die innere Ausnehmung des gesamten Halterungsschaftes 2 entformt werden kann.

Figur 5 zeigt im übrigen, daß die Kolbenstange 8 einen wesentlich geringeren Durchmesser hat als der lnnendurchmesser des Halterungschaftes 2, um hier ebenfalls einen Freiraum für das Zusammendrücken in Pfeilrichtung 19 zu schaffen.

Im praktischen Einsatz wird das gesamte Instrument auf dem Vorderteil eines endoskopischen Applikators aufgesetzt und dort verrastet. Es wird das gesamte Instrument in eine Hülse am Applikator zurückgezogen und der Applikator wird durch einen Trocar in den Körper eingeführt. Bei feststehender Hülse wird nun das Applikationsrohr mit dem am vorderen Ende befestigten Ende des befestigten Instrumentes in die Nähe des Operations- oder Behandlungsgebietes geschoben. Es wird nun die Zugstange des endoskopischen Applikators betätigt, wodurch die Kolbenstange 8 zurückgezogen wird und über die Saugöffnungen 16 im Deckel 12 eine entsprechende Saugwirkung auf das umgebende Körpergewebe ausgeübt wird, wodurch Körperflüssigkeit zu Untersuchungszwecken in der Zylinderkammer 15 aufgenommen wird. Es wird so dann das gesamte Instrument in die Hülse des endoskopischen Applikators zurückgeschoben, so daß es beim Herausziehen des Applikators aus dem Körper geschützt ist. Es wird dann sofort die Kappe 17 auf den Deckel 12 aufgesetzt, um ein Herauslaufen der aufgenommenen Körperflüssigkeit zu vermeiden.

### Zeichnungslegende

- 1.: Grundkörper
- 2.: Halterungsschaft
- 3.: Zylinderkörper
- 4.: Rastnase
- 5.: Anschrägung
- 6.: Schlitz
- 7.: Ring
- 8.: Kolbenstange
- 9.: Kolben
- 10.: Einhängung
- 11.: Öse
- 12.: Deckel
- 13.: Scheibe
- 14.: Schaft
- 15.: Zylinderkammer
- 16.: Saugöffnung
- 17.: Kappe
- 18.: Rand
- 19.: Pfeilrichtung
- 20: Entformungsöffnung
- 21.: Rastkante

## Patentansprüche

1. Endoskopisches Instrument zur Entnahme von Gewebsflüssigkeiten, **dadurch gekennzeichnet,** daß das endoskopische Instrument auswechselbar am vorderen Ende eines Applikationsrohres angeordnet ist und daß dieses endoskopische Instrument eine Kolbenstange (8) aufweist, die mit einer entsprechenden Zugstange eines endoskopischen Applikators koppelbar ist.

2. Endoskopisches Instrument zur Entnahme von Gewebsflüssigkeiten nach Anspruch 1, **dadurch gekennzeichnet,** daß an der Vorderseite der Zylinderkammer ein mit Saugöffnungen (16) ausgebildeter Deckel (12) angeordnet ist.

3. Endoskopisches Instrument zur Entnahme von Gewebsflüssigkeiten nach Anspruch 1 oder 2, **dadurch gekennzeichnet,** daß die Zylinderkammer (15) seitlich Saugöffnungen aufweist.

4. Endoskopisches Instrument zur Entnahme von Gewebsflüssigkeiten nach Anspruch 2 oder 3, **dadurch gekennzeichnet,** daß die Saugöffnungen (16) in distaler Richtung vor dem Kolben (9) sitzen.

5. Endoskopisches Instrument zur Entnahme von Gewebsflüssigkeiten nach Anspruch 1 - 4, **dadurch gekennzeichnet,** daß der Kolben (9) fest mit der Kolbenstange (8) verbunden ist.

6. Endoskopisches Instrument zur Entnahme von Gewebsflüssigkeiten nach Anspruch 1 - 5, **dadurch gekennzeichnet,** daß die gesamte Vorderseite der Zylinderkammer (15) mit dem dort bündig eingelassenen Deckel (12) mittels einer Kappe (17) verschließbar ist, wobei die Kappe (17) hierbei mindestens die Saugöffnungen (12) auslaufsicher abdeckt.
